# EUROPEAN PATENT APPLICATION

(11) **EP 4 502 605 A1**
(43) Date of publication of application: **05.02.2025**
(21) Application number: 23781337.3
(22) Date of filing: 29.03.2023
(51) Int. Cl.: G01N 33/574, C12Q 1/6886

(54) **NOVEL BIOMARKER FOR DETECTION OF CANCER METASTASIS**

(30) Priority: 30.03.2022 KR 20220039388
(71) Applicant: Industry-Academic Cooperation Foundation, Yonsei University, Seoul 03722 (KR)
(72) Inventor: GEE, Heon Yung, Seoul 03722 (KR); PARK, Hyun Woo, Seoul 03730 (KR); HUH, Hyunbin, Incheon 21017 (KR); SUB, Yujin, Seoul 03722 (KR)
(74) Representative: Müller, Christian Stefan Gerd
(86) International application number: PCT/KR2023/004157
(87) International publication number: WO 2023/191482

(57) **Abstract**

The present invention relates to agents for measuring the expression of proteins or genes encoding the proteins that are exclusively or specifically expressed in circulating tumor cells, or methods for preventing or treating cancer metastasis by controlling the expression of the proteins or the genes. The present invention provides a method for detecting cancer or cancer metastasis by identifying proteins or genes encoding proteins that are exclusively or specifically expressed in circulating tumor cells, and measuring their expression, and ultimately, may be effectively used for efficient prevention or treatment of cancer metastasis by inhibiting their expression.

## Description

### Technical Field

The present invention relates to a method for predicting cancer development or cancer metastasis by measuring the expression of a factor specifically expressed by a circulating tumor cell (CTC) in relation to the acquisition of metastatic ability of the cell, or a method for inhibiting cancer metastasis by regulating the expression of the factor.

### Background Art

Cancer is known to be the leading cause of death worldwide. In South Korea, cancer kills an average of 160 people per 100,000 people per year, making it the number one cause of death in the country (Cause of Death Statistics 2020). The vast majority of cancer patients die from metastasis of cancer rather than the primary tumor itself, which is the process where cancer cells break away from the primary tumor tissue and enter surrounding blood vessels or lymphatic vessels to form new tumors in other parts of the body. Numerically, more than 90% of cancer patients die as a result of metastasis from their primary cancer (Nature Reviews Cancer, 2006, 6:49-458). Therefore, early diagnosis of metastasis, or even predicting it before it occurs, is a critical issue with regard to improving the survival of cancer patients.

In the process in which tumor cells detach from the primary tumor and metastasize through the blood, the transition of primary tumor cells, which are adherent cells, to suspension cells, which are circulating tumor cells (CTCs), is necessary. A precise biomarker for these CTCs could be a useful indicator for diagnosing or predicting cancer metastasis.

Currently, many researches of cancer metastasis are hampered by the lack of a clear biomarker for CTCs, especially hematogenous cancer metastasis. The cells in the bloodstream that do not express CD45, a leukocyte-specific cell membrane protein, and express EpCAM, an epithelial cell-specific cell membrane protein, are defined as CTCs. These are almost the only marker currently used to discriminate CTCs in research and clinical practice. Although the role of EpCAM is still unclear, EpCAM is expressed on the cell membrane of CTCs and is not lost during the epithelial-mesenchymal transition (EMT). However, single-cell transcriptome analysis of EpCAM expression in a mouse model revealed that EpCAM is expressed in only 41.0% of CTCs. These results suggest that EpCAM is detected in less than half of CTCs, therefore using EpCAM as a marker to isolate CTCs will miss a significant number of EpCAM-negative CTCs.

Accordingly, in order to discover an efficient biomarker for the early detection or prediction of the progression of primary cancer to cancer metastasis, the present inventors studied the association of specific genes exclusively expressed in CTCs with the progress of metastasis, and confirmed that said genes plays an crucial role in diagnosing or predicting the progression of cancer cells to metastasis.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop a diagnostic method that can detect cancer metastasis at an early stage or predict it at a pre-metastatic stage, thereby significantly reduce the mortality rate of cancer metastasis, by identifying novel biomarkers that improve the low accuracy of conventional biomarkers for cancer metastasis. As a result, the present inventors have discovered six proteins that are exclusively expressed on the cell membrane of circulating tumor cells (CTCs), confirmed their potential as biomarkers for CTCs, and found that these biomarkers can be used to predict the progression of primary cancer cells to the metastatic stage at an early stage with high accuracy, thereby completing the present invention.

Accordingly, it is an object of this invention to provide a diagnostic composition capable of predicting whether cancer will metastasize.

It is another object of this invention to provide a composition for detecting a Circulating Tumor Cells.

It is still another object of this invention to provide a kit for diagnosing a cancer or cancer metastasis.

It is still another object of this invention to provide a method for providing information necessary for the diagnosis of cancer or cancer metastasis.

It is still another object of this invention to provide a composition for preventing or treating cancer metastasis.

It is still another object of this invention to provide a method for screening a composition for the preventing or treating cancer metastasis.

Other objects and advantages of the present invention will become more apparent from the following detailed description, the appended claims, and the accompanying drawings.

### Technical Solution

In one aspect of this invention, there is provided a composition for diagnosing a cancer comprising, as an active ingredient, an agent for measuring the expression of at least one protein selected from the group consisting of AFDN, CDC42EP1, EPHA2, GPRC5A, JPT2 and NIBAN2, or a gene encoding the protein

The present inventors have made intensive studies to develop a diagnostic method that can detect cancer metastasis at an early stage or predict it at a pre-metastatic stage, thereby significantly reduce the mortality rate of cancer metastasis, by identifying novel biomarkers that improve the low accuracy of conventional biomarkers for cancer metastasis. As a result, the present inventors have discovered six proteins that are exclusively expressed on the cell membrane of circulating tumor cells (CTCs), confirmed their potential as biomarkers for CTCs, and found that these biomarkers can be used to predict the progression of primary cancer cells to the metastatic stage at an early stage with high accuracy, thereby completing the present invention.

The term "diagnosis" as used herein includes determination of the susceptibility of a subject to a particular disease, determination of whether a subject currently has a particular disease, and determination of the prognosis of a subject suffering from a particular disease.

The term "composition for diagnosing" as used herein refers to an integrated mixture or device including a means for detecting the expression level of the AFDN, CDC42EP1, EPHA2, GPRC5A, JPT2 or NIBAN2 protein or the *AFDN, CDC42EPI, EPHA2, GPRC5A, JPT2* or *NIBAN2* gene in order to determine whether tumor cells in a subject have acquired metastatic ability or are likely to acquire metastatic ability. Therefore, the term may also be expressed as a "kit for diagnosing".

The term "AFDN" as used herein refers to a protein named Afadin encoded by the *AFDN* gene, which may also be referred as AF6, MLL-AF6, MLLT4, myeloid/lymphoid or mixed-lineage leukemia; translocated to, 4, afadin, adherens junction formation factor and 1-afadin.

The term "CDC42EP1" as used herein refers to a protein named Cdc42 effector protein 1 encoded by the *CDC42EP1* gene, which may also be referred as BORG5, CEP1, MSE55, CDC42 effector protein 1.

The term "EPHA2" as used herein refers to a protein named EPH receptor A2 or ephrin type-A receptor 2 encoded by the *EPHA2* gene, which may also be referred as Epha2, AW545284, Eck, Myk2, Sek-2, Sek2, ARCC2, CTPA, CTPP1, CTRCT6, EPH receptor A2 and ECK.

The term "GPRC5A" as used herein refers to a protein named Retinoic acid-induced protein 3 encoded by the *GPRC5A* gene which may also be referred as GPCR5A, RAI3, RAIG1, PEIG-1, TIG1 and G protein-coupled receptor class C group 5 member A.

The term "JPT2" as used herein refers to a protein named Hematologic and neurological expressed 1-like protein encoded by the *HN1L* gene which may also be referred as C16orf34, L11, HN1L, hematologic and neurological expressed 1-like, hematologic and neurological expressed 1 like and Jupiter microtubule associated homolog 2.

The term "NIBAN2" as used herein refers to a protein named Niban Apoptosis regulator 2 encoded by the NIBAN2 gene.

According to a specific embodiment, the cancer is a metastatic cancer.

The term "metastatic cancer" as used herein refers to a new tumor formed when tumor cells detached from the primary tumor tissue invade the surrounding blood vessels or lymphatic vessels and migrate to other distal parts of the body through these vessels as passages. Since more than 90% of cancer patient deaths are attributable to metastasis from the primary tumor (Nature Reviews Cancer, 2006, 6:449-458), suppressing metastasis to reduce the mortality of cancer patients is as important as treating primary cancer.

The mechanism by which tumor cells acquire mobility during the metastatic process is explained by an epithelial-to-mesenchymal transition (EMT), in which tumor epithelial cells acquire the characteristics of mesenchymal cells through genetic mutation, and a mesenchymal-to-epithelial transition (MET), which is the opposite process (J Clin Invest. 2009, 119:1417-1419). In other words, epithelial cells that have the characteristics of mesenchymal cells detach from their original location due to their weak intercellular adhesion and migrate to blood vessels, and the cells that have been migrating through blood vessels regain their original epithelial characteristics (MET) and settle at secondary sites distal to the primary site, causing a tumor to form.

The present inventors have identified genes that are specifically and exclusively expressed in circulating tumor cells (CTCs), and found that these genes may function as reliable biomarkers for the diagnosis of cancer metastasis or for predicting the progression of a primary cancer to metastasis. Therefore, if said biomarkers are highly expressed, it could be determined that circulating tumor cells that cause metastatic cancer have already been generated or are at high risk of being generated in the future. Therefore, the term "diagnosis of metastatic cancer" is used interchangeably with "diagnosis of cancer metastasis", "prediction of epithelial-mesenchymal transition", "prediction of circulating tumor cell generation" or "prediction of cancer prognosis".

According to a specific embodiment, the composition comprises an agent for measuring expression of the CDC42EP1 or NIBAN2 protein or the gene encoding the protein.

According to a specific embodiment, the agent for measuring the expression of a gene encoding the at least one protein selected from the group consisting of AFDN, CDC42EP1, EPHA2, GPRC5A, JPT2 and NIBAN2 is a primer or probe that specifically binds to a nucleic acid molecule of the gene.

The term "nucleic acid molecule" as used herein is meant to encompass DNA (gDNA and cDNA) and RNA molecules. Nucleotides, which are the basic structural units in nucleic acid molecules, include not only natural nucleotides, but also analogues having modified sugar or base moieties (Scheit, Nucleotide Analogs, John Wiley, New York (1980); Uhlman and Peyman, Chemical Reviews, 90:543-584 (1990)).

The term "primer" as used herein refers to an oligonucleotide which serves as a starting point for synthesis under conditions in which the synthesis of a primer extension product complementary to a nucleic acid chain (template) is induced, i.e., the presence of nucleotides and a polymerase such as DNA polymerase, and suitable temperature and pH. Specifically, the primer is a deoxyribonucleotide single chain. The primers that are used in the present invention may include naturally occurring dNMPs (i.e., dAMP, dGMP, dCMP and dTMP), modified nucleotides or non-natural nucleotides. In addition, the primers may also include ribonucleotides.

The primer of the present invention may be an extension primer which is annealed to a target nucleic acid and forms a sequence complementary to the target nucleic acid by a template-dependent nucleic acid polymerase. The extension primer is extended to the location where an immobilized probe is annealed and occupies the area at which the probe is annealed.

The extension primer used in the present invention includes a hybridizing nucleotide sequence complementary to a specific nucleotide sequence of a target nucleic acid, e.g., the *AFDN, CDC42EP1, EPHA2, GPRC5A, JPT2,* or *NIBAN2* gene. The term "complementary" means that a primer or a probe is complementary enough to selectively hybridize to a target nucleic acid sequence under a certain annealing or hybridization condition. The term "complementary" encompasses both "substantially complementary" and "perfectly complementary", and specifically, it means perfectly complementary. In the present specification, the term "substantially complementary sequence" is meant to include not only a completely matching sequence, but also a sequence partially mismatching with the sequence to be compared within a range where it can anneal to a particular sequence and serve as a primer.

The primer should be long enough to prime the synthesis of an extension product in the presence of a polymerase. The suitable length of the primer is determined depending on a number of factors, such as temperature, pH and the source of the primer, but is typically 15 to 30 nucleotides. Short primer molecules generally require lower temperatures to form a sufficiently stable hybrid complex with a template. The design of such a primer can be easily carried out by those skilled in the art with reference to a target nucleotide sequence using, for example, a program for primer design (e.g., PRIMER 3 program).

The term "probe" refers to a natural or modified monomer or a linear oligomer having linkages, which includes a deoxyribonucleotide and ribonucleotide that can be hybridized with a specific nucleotide sequence. Specifically, the probe is single-stranded for maximum efficiency in hybridization. More specifically, the probe is a deoxyribonucleotide. As the probe that is used in the present invention, a sequence perfectly complementary to a specific nucleotide sequence of the *AFDN, CDC42EP1, EPHA2, GPRC5A, JPT2,* or *NIBAN2* gene may be used, but a sequence substantially complementary thereto may also be used as long as specific hybridization is not interrupted. In general, the stability of a duplex formed by hybridization tends to be determined by the match of terminal sequences, and thus it is preferred to use a probe which is complementary to the 3'- end or 5'-end of the target sequence.

Conditions suitable for hybridization may be determined with reference to the contents disclosed in Joseph Sambrook, et al., Molecular Cloning, A Laboratory Manual, Cold Spring Harbor Laboratory Press, N.Y. (2001), and Haymes, B. D., et al., Nucleic Acid Hybridization, A Practical Approach, IRL Press, Washington, D.C. (1985).

According to a specific embodiment, the agent for measuring the expression of the at least one protein selected from the group consisting of AFDN, CDC42EP1, EPHA2, GPRC5A, JPT2, and NIBAN2 is an antibody or an antigen-binding fragment thereof that specifically binds to the at least one protein selected from the group consisting of AFDN, CDC42EP1, EPHA2, GPRC5A, JPT2 and NIBAN2; or an aptamer that specifically binds to the at least one protein selected from the group consisting of AFDN, CDC42EP1, EPHA2, GPRC5A, JPT2, and NIBAN2.

As used herein, the term "antibody" refers to a molecule that specifically binds to an antigen and causes an antigen-antibody interaction. For the purposes of the present invention, the antibody refers to one that binds specifically to the polypeptides recited herein.

According to the present invention, the AFDN, CDC42EP1, EPHA2, GPRC5A, JPT2 or NIBAN2 protein of the present invention may be detected by an immunoassay method using an antigen-antibody reaction, thereby analyze whether tumor cells have acquired metastatic ability. This immunoassay may be performed according to various conventional immunoassay or immunostaining protocols.

For example, when the method of the present invention is performed according to a radioimmunoassay method, antibodies labeled with radioisotopes (e.g., C¹⁴, I¹²⁵, P³², and S³⁵) may be used. In the present invention, the antibody that specifically recognizes the AFDN, CDC42EP1, EPHA2, GPRC5A, JPT2 or NIBAN2 protein is a polyclonal or monoclonal antibody, preferably a monoclonal antibody.

The antibody of the present invention may be produced by methods commonly practiced in the art, for example, the fusion method (Kohler and Milstein, European Journal of Immunology, 6:511-519 (1976)), the recombinant DNA method (US Patent No. 4,816,567), or the phage antibody library method (Clackson et al, Nature, 352:624-628 (1991), and Marks et al, J. Mol. Biol., 222:58, 1-597 (1991)). General procedures for antibody production are described in detail in Harlow, E. and Lane, D., Using Antibodies: A Laboratory Manual, Cold Spring Harbor Press, New York, 1999; and Zola, H., Monoclonal Antibodies: A Manual of Techniques, CRC Press, Inc., Boca Raton, Florida, 1984.

By analyzing the intensity of the final signal by the above-described immunoassay process, it is possible to predict whether a tumor has metastasized or is likely to metastasize. In other words, if the signal for the AFDN, CDC42EP1, EPHA2, GPRC5A, JPT2 or NIBAN2 protein in a sample from a subject is stronger than that in a normal sample, it is determined that the tumor in the subject has metastasized or is highly likely to metastasize in the future.

The term "antigen-binding fragment" as used herein refers to a portion of a polypeptide, to which an antigen can bind, among the entire structure of an immunoglobulin. Examples of the antigen-binding fragment include, but are not limited to, F(ab')2, Fab', Fab , Fv, and scFv.

The term "specifically binding" as used herein has the same meaning as "specifically recognizing", and means that an antigen and an antibody (or a fragment thereof) specifically interact with each other by an immunological reaction.

In the present invention, an aptamer that specifically binds to AFDN, CDC42EP1, EPHA2, GPRC5A, JPT2 or NIBAN2 protein may be used instead of an antibody. In the present specification, the term "aptamer" refers to a single-stranded nucleic acid (RNA or DNA) molecule or peptide molecule that binds to a specific target substance with high affinity and specificity. General contents regarding aptamers are disclosed in detail in Hoppe-Seyler F, Butz K "Peptide aptamers: powerful new tools for molecular medicine". J Mol Med. 78(8):426-30(2000); Cohen BA, Colas P, Brent R. "An artificial cell-cycle inhibitor isolated from a combinatorial library". Proc Natl Acad Sci USA. 95(24):14272-7(1998).

According to a specific embodiment, the cancer is breast cancer, ovarian cancer, or colon cancer.

In another aspect of this invention, there is provided a composition for detecting a Circulating Tumor Cell comprising, as an active ingredient, an agent for measuring the expression of at least one protein selected from the group consisting of AFDN, CDC42EP1, EPHA2, GPRC5A, JPT2 and NIBAN2, or a gene encoding the protein.

The term "detect" as used herein refers to determine whether a target substance is present in a confined space, wherein "confined space" includes, but is not limited to, a target organism, sample, reaction mixture, molecular complex, or substrate. Specifically, the term "detection" includes any act by which a person of ordinary skill in the art can determine the chemical or biological properties of a target substance, based on its ability to interact, bind with other compounds, activate other compounds, or any other additional characteristic.

Detection can be quantitative or qualitative. "Quantitative" detection refers to measuring the amount of a target substance or signal. The measurement method includes any method that measures the amount of a target substance or the intensity of a signal. "Qualitative" detection refers to detecting the presence of a target substance, which is not quantified, through its relative abundance to other substances.

The term "circulating tumor cell" as used herein, also referred to as a CTC, refers to a tumor cell that breaks away from a primary tumor or a tumor that has metastasized from a primary tumor, enters a blood vessel, and circulates with the bloodstream. Circulating tumor cells can transform back into adherent cells at sites far from the primary site, giving rise to new cancer metastases.

According to the present invention, CTCs can be detected in biological samples such as blood, serum, plasma or body fluids using the biomarkers of the present invention, and the detection of these CTCs can be achieved through a non-invasive "liquid biopsy". Compared to traditional biopsies, liquid biopsies have the advantage of being applicable to most patients and easy to collect (Front Oncol. 11: 652253, 2021).

Examples of diagnosing cancer or cancer metastasis through the detection of CTCs as described above include colorectal cancer *(*Biomarker Research volume 9, Article number: 85, 2021), gastric cancer (J Cancer Metastasis Treat ;4:32., 2018), or breast cancer *(*Front Oncol. 11: 652253, 2021). Considering the mechanism of action of cancer metastasis where the circulating tumor cells are produced, it may be applicable to any type of cancer.

Because CTCs are known to be statistically significantly associated with a cancer patient's stage, tumor size, and whether the cancer cells are invasive or have spread to the lymph nodes *(*Cancer Med. 2020 Mar; 9(5): 1638-1647), in addition to diagnosing cancer or cancer metastasis, the staging, tumor size, invasiveness, etc. can be predicted by detecting CTCs.

Accordingly, the term "circulating tumor cell detection" in the present invention encompasses diagnosis of cancer or cancer metastasis, prediction of metastasis, staging, diagnosis of tumor size, and prediction of invasiveness of cancer cells.

In still another aspect of this invention, there is provided a kit for diagnosing a cancer comprising above mentioned composition.

According to the present invention, the kit may be, but is not limited to, an RT-PCR kit, a DNA chip kit, an ELISA kit, a protein chip kit, a rapid kit, or a multiple reaction monitoring (MRM) kit.

The kit for the diagnosis of cancer metastasis may further comprise one or more other component compositions, solutions or devices suitable for the assay method.

For example, the kit for diagnosis of cancer may further comprise essential elements necessary to perform a reverse transcription polymerase reaction. The reverse transcription polymerase reaction kit includes a pair of primers specific for a gene encoding a marker protein. The primers are nucleotides having a sequence specific to the nucleic acid sequence of said gene, and may have a length of from about 7 bp to 50 bp, more preferably from about 10 bp to 30 bp. It can also include a primer specific for a nucleic acid sequence of a control gene. In addition, the kit may include a test tube or other suitable container, reaction buffer (pH and magnesium concentration vary), deoxynucleotides (dNTPs), enzymes such as Taq-polymerase and reverse transcriptase, DNase, RNase inhibitor DEPC-water and sterile water.

Further, the kit of the present invention may include the essential elements required to perform a DNA chip. A DNA chip kit may include a substrate to which a cDNA or oligonucleotide corresponding to a gene or fragment thereof is attached, and reagents, agents, enzymes, etc. for making a fluorescently labeled probe. The substrate can also include a cDNA or oligonucleotide corresponding to a control gene or a fragment thereof.

Furthermore, the kit of the present invention may comprise the essential elements required to perform an ELISA. The ELISA kit includes an antibody specific for said protein. The antibody may be a monoclonal antibody, polyclonal antibody, or recombinant antibody that has high specificity and affinity for the marker protein and little cross-reactivity to other proteins. The ELISA kit may also include an antibody specific for a control protein. In addition, ELISA kits can include reagents to detect bound antibodies, such as labeled secondary antibodies, chromophores, enzymes (e.g., conjugated with antibodies) and their substrates, or other substances that can bind to antibodies.

In the diagnostic kit of the present invention, the immobilizers for antigen-antibody binding reactions can be used, but are not limited to, nitrocellulose membranes, PVDF membranes, well plates synthesized from polyvinyl resin or polystyrene resin, and glass slide glasses.

Furthermore, in the kits of the present invention, the labeling agent for the secondary antibody is preferably a conventional chromogen, including, but not limited to, horseradish peroxidase (HRP), alkaline phosphatase, colloidal gold, fluorescein, and dyes such as FITC (poly-L-lysine-fluorescein isothiocyanate) and RITC (rhodamine-B-isothiocyanate).

The chromogenic substrate for inducing color development in the diagnostic kit of the present invention is preferably used according to the chromogenically reacting marker, and can be TMB (3,3',5,5'-tetramethylbenzidine), ABTS [2,2'-azino-bis(3-ethylbenzothiazoline-6-sulfonic acid)], OPD (o-phenylenediamine), and the like. Preferably, the chromogenic substrate is provided dissolved in a buffer solution (0.1 M NaAc, pH 5.5). The chromogenic substrate, such as TMB, is digested by HRP used as a label for the secondary antibody conjugate, to generate a chromogenic precipitate, and the presence or absence of the marker protein is visually determined by the extent of the chromogenic precipitate.

In the diagnostic kit of the present invention, the wash solution preferably comprises phosphate buffer, NaCl, and Tween 20, and more preferably a buffer solution (PBST) comprising 0.02 M phosphate buffer, 0.13 M NaCl, and 0.05% Tween 20. The wash solution is prepared by reacting the antigen-antibody conjugate with secondary antibody after the antigen-antibody binding reaction, and then adding an appropriate amount to the fixative for three to six washes. Sulfuric acid solution (H₂SO₄) is preferably used as the reaction stop solution.

According to a specific embodiment, the cancer is a metastatic cancer.

The metastatic cancer involved in the present invention has already been described above in detail and is therefore omitted to avoid undue redundancy.

In still another aspect of this invention, there is provided a method for providing information necessary for the diagnosis of cancer, comprising measuring the expression of at least one protein selected from the group consisting of AFDN, CDC42EP1, EPHA2, GPRC5A, JPT2 and NIBAN2, or genes encoding the protein, in a biological sample isolated from a subject.

The AFDN, CDC42EP1, EPHA2, GPRC5A, JPT2, and NIBAN2 proteins of the present invention, the genes encoding them, and the cancers to be diagnosed have already been described above in detail and are be omitted to avoid undue redundancy.

The present inventors have discovered for the first time that the expression level of AFDN, CDC42EP1, EPHA2, GPRC5A, JPT2 or NIBAN2 protein and the acquisition of metastatic ability by tumor cells have a positive correlation. Accordingly, it is determined that, when the IZFK1, IKZF3, NFE2 or IRF8 protein or the gene encoding the same is highly expressed in a subject, the subject has tumor cells with metastatic ability or will have in the future.

The term "highly expressed" as used herein means that the expression level of the protein or gene is significantly higher than that in a control group in which metastasis has not occurred or the likelihood of metastasis is low. Specifically, the term means that the expression level is 130% or more, more specifically 150% or more, most specifically 170% or more, of that in the control.

The term "subject" as used herein refers to an individual that provides a sample in which the expression level of the AFDN, CDC42EP1, EPHA2, GPRC5A, JPT2 or NIBAN2 protein or the gene encoding the same is to be measured, and which is ultimately to be analyzed as to the acquisition of metastatic ability by tumor cells. The term "subject" includes, without limitation, humans, mice, rats, guinea pigs, dogs, cats, horses, cows, pigs, monkeys, chimpanzees, baboons or rhesus monkeys. Specifically, the subject is a human. Since the composition of the present invention provides information on whether tumor cells have acquired metastatic ability, but also information for predicting whether tumor cells have a genetic risk of acquiring metastatic ability in the future, the subject of the present invention may be a metastatic cancer patient or a patient with primary cancer that has not yet acquired metastatic ability.

According to a specific embodiment, the cancer is a metastatic cancer.

The metastatic cancer involved in the present invention has already been described above in detail and is therefore omitted to avoid undue redundancy.

According to a specific embodiment, the biological sample is selected from the group consisting of whole blood, plasma and serum.

The term "whole blood" as used herein generally refers to blood consisting of unclotted plasma and cellular components. Plasma may comprise about 50 to 60% of the volume of whole blood, and about 40 to 50% of cellular components (e.g., red blood cells, white blood cells, or platelets.

The term "plasma" as used herein refers to the liquid component of blood, which functions as a transport medium to deliver nutrients to the body's cells and organs.

The term "serum" as used herein refers to the pale yellow liquid collected from blood and, more specifically, to the pale yellow fluid component that remains when blood is collected and left to stand, which, when left unattended, reduces the fluidity of the blood and causes red clots to form, and which remains when the red clots are removed.

According to the present invention, the biomarkers of the present invention may be used to diagnose cancer or cancer metastasis, by liquid biopsy using patient-derived fluids, such as blood, which is advantageous of being non-invasive compared to conventional tissue biopsy procedures, minimizing patient suffering, and providing clinical information more quickly.

According to a specific embodiment, the cancer is breast cancer, ovarian cancer, or colon cancer.

In still another aspect of this invention, there is provided a composition for preventing or treating cancer metastasis comprising, as an active ingredient, an inhibitor of at least one protein selected from the group consisting of AFDN, CDC42EP1, EPHA2, GPRC5A, JPT2 and NIBAN2, or a gene encoding the protein.

The term "inhibitor" as used herein refers to a substance that reduces the activity or expression of a target protein or gene not only where the activity or expression becomes undetectable or is present at insignificant levels, but also where the activity or expression is decreased to such an extent that the biological function of the target protein or gene is significantly impaired.

Examples of the inhibitor of the target gene include, but are not limited to, shRNA, siRNA, miRNA, ribozyme, peptide nucleic acid (PNA), and an antisense oligonucleotide, which inhibit the expression of the gene whose sequence is already known in the art at the gene level; a CRISPR system comprising a guide RNA that recognizes the target gene; antibodies or aptamers that inhibit the expression at the protein level; and compounds, peptides and natural products that inhibit the activity of the target gene. In addition, any means for gene and protein level inhibition known in the art may be used.

The term "small hairpin RNA (shRNA)" as used herein refers to a single-stranded RNA sequence consisting of 50 to 70 nucleotides, which forms a stem-loop structure *in vivo* and creates a tight hairpin structure for inhibiting target gene expression by RNA interference. Usually, a double-stranded stem is formed by complementary base pairing of a long RNA consisting of 19 to 29 nucleotides on both sides of the loop consisting of 5 to 10 nucleotides, and for constitutive expression, the double-stranded stem is transduced into cells via a vector including U6 promoter and is usually delivered to daughter cells so that inhibition of expression of the target gene is inherited.

The term "siRNA" as used herein refers to a short double-stranded RNA capable of inducing RNA interference (RNAi) through cleavage of a specific mRNA. The siRNA is composed of a sense RNA strand having a sequence homologous to the mRNA of the target gene and an antisense RNA strand having a sequence complementary thereto. The total length thereof may be 10 to 100 bases, preferably 15 to 80 bases, most preferably 20 to 70 bases, and the ends thereof may be blunt or cohesive as long as the siRNA is capable of inhibiting the expression of the target gene by the RNAi effect. The cohesive ends may have a 3' end overhang and a 5" end overhang.

The term "microRNA (miRNA)" as used herein refers to an oligonucleotide that is not expressed in cells, and means a single-stranded RNA molecule that inhibits target gene expression by complementary binding to the mRNA of the target gene while having a short stem-loop structure.

The term "ribozyme" as used herein refers to an RNA molecule with enzyme-like functionality that recognizes a specific sequence of RNA and cleaves it by itself. A ribozyme consists of a region that binds with specificity to complementary sequences in the target mRNA strand and a region that cleaves the target RNA.

The term "peptide nucleic acid (PNA)" as used herein refers to a molecule capable of complementary binding to DNA or RNA while having both nucleic acid and protein properties. PNA is not found in nature, is artificially synthesized by a chemical method, and regulates target gene expression by forming a double strand through hybridization with a natural nucleic acid having a nucleotide sequence complementary thereto.

The term "antisense oligonucleotide" as used herein refers to a nucleotide sequence complementary to a specific mRNA sequence. Specifically, it refers to a nucleic acid molecule which binds to a complementary sequence in a target mRNA and inhibits the activities of the target mRNA, which are essential for translation into proteins, translocation into cytoplasm, maturation, or all other overall biological functions. The antisense oligonucleotide may be modified at one or more bases, sugars or backbone positions to enhance efficacy thereof (De Mesmaeker et al., Curr Opin Struct Biol., 5 (3) : 343-55, 1995). The oligonucleotide backbone may be modified with phosphorothioate, phosphotriester, methylphosphonate, short-chain alkyl, cycloalkyl, short-chain heteroatomic, heterocyclic sugar sulfonate, or the like.

The term "guide RNA (gRNA)" as used herein refers to an RNA molecule used in a gene editing system that recognizes a target gene and induces a nuclease to specifically cleave the recognized site. Typical examples of this gene editing system include a CRISPR (Clustered Regularly Interspaced Short Palindromic Repeats) system.

According to the present invention, the inhibitor of the present invention may be an antibody that inhibits the activity of the protein encoded by the gene. An antibody specifically recognizing the target protein is a polyclonal or monoclonal antibody, preferably a monoclonal antibody.

The antibodies and aptamers involved in the present invention have already been described above in detail and are therefore omitted to avoid undue redundancy.

The term "preventing" as used herein refers to inhibiting the occurrence of a disorder or disease in a subject who has never been diagnosed as having the disorder or disease, but is at risk of developing the condition or disease.

The term "treating" as used herein refers to (a) inhibiting the progress of a disorder, disease or symptom; (b) alleviating the disorder, disease or symptom; or (c) eliminating the disorder, disease or symptom. When the composition of the present invention is administered to a subject, it functions to inhibit the generation of circulating tumor cells while inhibiting the expression of the AFDN, CDC42EP1, EPHA2, GPRC5A, JPT2, NIBAN2 protein or the gene encoding the same, thereby inhibiting, eliminating, or alleviating the progress of symptoms resulting from a tumor, specifically a metastatic tumor. Thus, the composition of the present invention may serve as a therapeutic composition for these diseases by itself, or may be administered in combination with other pharmacological ingredients and applied as a therapeutic aid for the diseases. Accordingly, as herein used, the term "treatment" or "therapeutic agent" is meant to encompass "treatment aid" or "therapeutic adjunct".

In still another aspect of this invention, there is provided a method for screening a composition for preventing or treating metastatic cancer, comprising:
(a) contacting a test substance with a biological sample comprising at least one protein selected from the group consisting of AFDN, CDC42EP1, EPHA2, GPRC5A, JPT2 and NIBAN2, a gene encoding the protein, or a cell expressing the protein or gene; and
(b) measuring the expression of the at least one protein selected from the group consisting of AFDN, CDC42EP1, EPHA2, GPRC5A, JPT2 and NIBAN2, or the gene encoding the protein, in the biological sample,

wherein, when the expression level of the protein or the gene in the biological sample decreases, the test substance is determined to be a composition for preventing or treating cancer metastasis.

The factors that are specifically and exclusively expressed in circulating tumor cells used and the types of cancer to be prevented or treated by modulating their expression have already been described above in detail and are therefore omitted to avoid undue redundancy.

The term "biological sample" as used herein refers to any sample containing cells expressing the above-described gene, which is obtained from mammals, including humans. Examples of the biological sample include, but are not limited to, tissues, organs, cells, or cell cultures. More specifically, the biological sample may be tumor tissue, tumor cells, or a culture thereof.

The term "test substance" as used while referring to the screening method of the present invention refers to a material which is used in screening to examine whether it affects the activity or expression level of the protein or gene of the present invention by being added to a sample containing cells expressing the gene. Examples of the test substance include, but are not limited to, compounds, nucleotides, peptides, and natural extracts. The step of measuring the expression level or activity of the gene in the biological sample treated with the test substance may be performed by various expression level and activity measurement methods known in the art.

According to a specific embodiment, the biological sample contains tumor tissue or tumor cells.

In still another aspect of this invention, there is provided a method for diagnosing cancer metastasis comprising administering, to a subject, a composition for diagnosing metastatic cancer comprising, as an active ingredient, an agent for measuring the expression of at least one protein selected from the group consisting of AFDN, CDC42EP1, EPHA2, GPRC5A, JPT2 and NIBAN2, or a gene encoding the protein.

In still another aspect of this invention, there is provided a method for preventing or treating metastatic cancer comprising administering, to a subject, a composition for preventing or treating cancer metastasis comprising, as an active ingredient, an inhibitor of at least one protein selected from the group consisting of AFDN, CDC42EP1, EPHA2, GPRC5A, JPT2 and NIBAN2, or a gene encoding the protein.

Agents for measuring the expression of the at least one protein selected from the group consisting of AAFDN, CDC42EP1, EPHA2, GPRC5A, JPT2, and NIBAN2, or genes encoding them, inhibitors thereof, and cancers to be diagnosed or treated by inhibiting said genes have already been described above in detail and are therefore omitted to avoid undue redundancy.

### Advantageous Effects

The features and advantages of the present invention are summarized as follows:
(a) The present invention provides a method for preventing or treating cancer metastasis by modulating the expression of a protein or gene encoding the protein that is exclusively or specifically expressed in circulating tumor cells.
(b) The present invention provides methods for detecting cancer or cancer metastasis by identifying proteins or genes encoding the proteins that are exclusively or specifically expressed in circulating tumor cells and measuring their expression. Thus, the present invention may be useful for efficient prevention or treatment of metastatic cancer by inhibiting the expression of these factors.

### Brief Description of Drawings

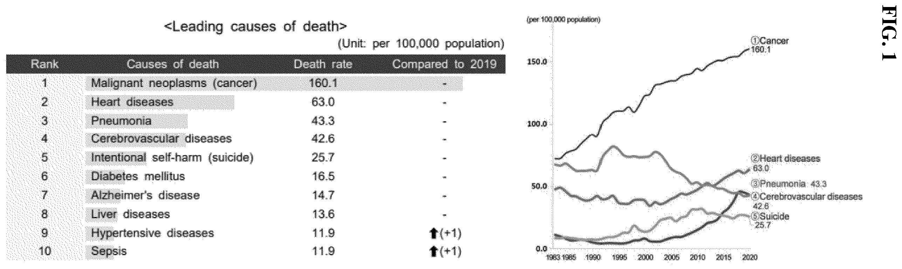

FIG. 1 represents a graph from Statistics Korea, showing that cancer has been the number one cause of death since 1990, with 160.1 deaths per 100,000 people in 2020.

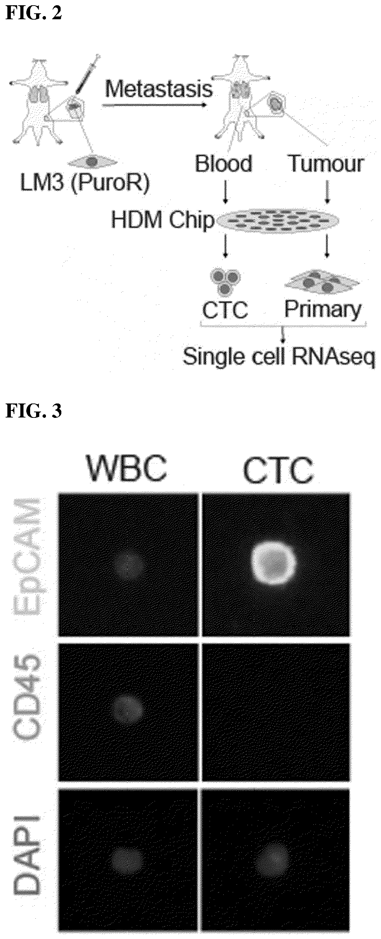

Figure 2 is a schematic illustration of the single-cell transcriptome analysis process for primary cancer cells and circulating tumor cells, using a mouse model with xenografted cancer cell lines and a high-density micropore chip (HDM Chip).
Figure 3 shows the identification of EpCAM+/CD45- circulating tumor cells and EpCAM-/CD45+ leukocytes in blood vessels using immunofluorescence.
Figure 4 represents UMAP maps, Feature Plots and Violin Plots for primary cancer cells and circulating tumor cells, where the plots shows the expression of EpCAM and CD45 transcripts in primary cancer cells and circulating tumor cells.
FIG. 5 shows the number of circulating tumor cells that are EpCAM+/CD45-, based on single cell transcriptome analysis.
Figure 6 shows a shortlist of eight biomarker candidates that are exclusively expressed in metastatic cancers and not in leukocytes, with the selection criteria being proteins expressed in more than 50% of circulating tumor cells and less than 1% of leukocytes, excluding MET and BCAR1, which are widely recognized markers of breast cancer.

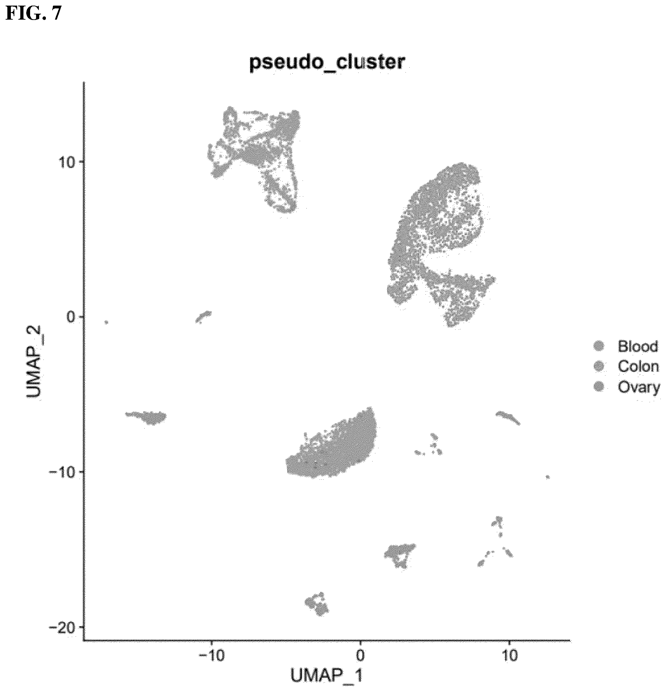

Figure 7 represents UMAP maps of tumor cells derived from ovarian cancer patients, colon cancer patients, and healthy controls.

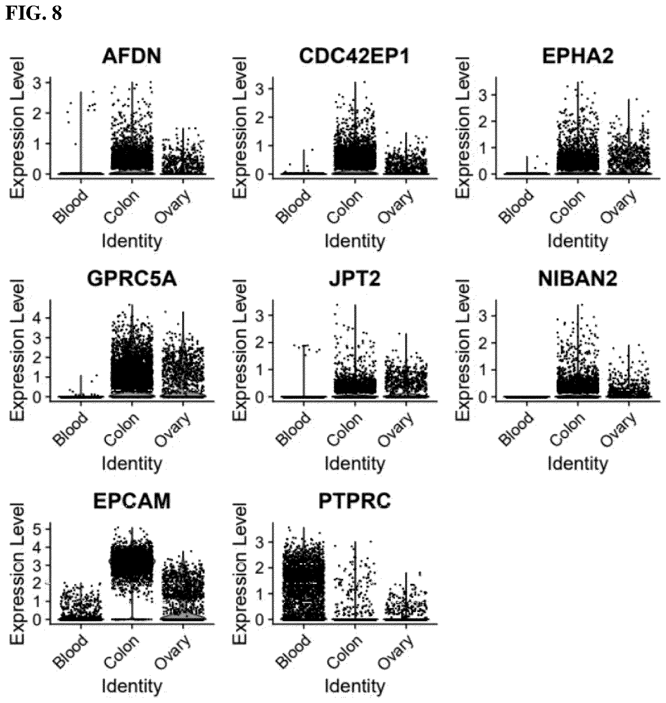

Figure 8 shows the six novel biomarker candidates and a Violin Plot of EpCAM and CD45 (PTPRC), showing that the six novel biomarkers are more exclusively expressed on circulating tumor cells than EpCAM, a conventional CTC biomarker.

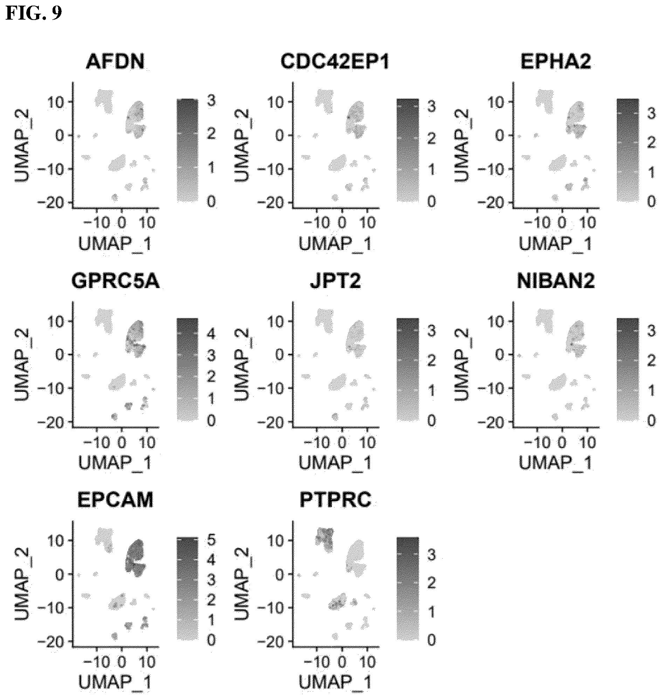

Figure 9 shows the feature plots of the six novel biomarker candidates and EpCAM and CD45 (PTPRC).

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Examples

### Experimental Methods and Analysis Methods

### CDX models and animal testing

The breast cancer cell line LM3 was kindly provided by Dr. H.W Park, Department of Biomedical Engineering, College of Biosystems, Yonsei University. Cultured LM3-GFP xenografts were performed after removal of mammary fat pads from immunocompromised 3-4 week old NOD/SCID or NGS mice according to established procedures. Tumor growth and metastasis were monitored weekly. When tumors reached 2.0-2.5 cm in length or thickness, the animals were euthanized, and tissue and blood were collected.

### Tissue collection and isolation

The animals were euthanized by cervical dislocation after asphyxiation using CO₂ and blood samples were extracted from the heart via syringe. After the blood samples were prepared, the mice were treated with EDTA (D-PBS solvent) at a concentration of 10 mM. Primary tumors of mice were mechanically cut for flow cytometry and then treated with collagenase IV (collagenase IV, Sigma-Aldrich cat.no.C5138-1G) in solvent (RPMI-F12 medium with 5% FBS, 5 µg ml-1 insulin and 1% penicillin/streptomycin solution) at 37°C for 45 minutes. Cell suspensions were washed with 2 µg ml-1 DNAse I (Worthington Biochemical, cat. no. LS002139) for 5 min and further separated with 0.05% trypsin for 10 min. The cells were then washed with Hanks balanced salt solution with 2% PBS, and the cells were passed through a 70-µm filter. To remove red blood cells by lysis, primary tumor cells and blood cells were treated with 1 X RBC lysis buffer and then resuspended in PBS with 2% FBS for immunofluorescence assays and cell separation.

### Immunofluorescence Assays and Imaging

To measure epithelial marker (EpCAM) and leukocyte marker (CD45) on the membrane surface of circulating tumor cells, cells were attached to slides by cell centrifugation. To perform immunofluorescence, slides were incubated overnight at 4°C with antibodies against EpCAM (Abcam, ab213500, 1:100 dilution) and antibodies against CD45 (Abcam, ab33923, 1:100 dilution), followed by incubation with fluorescent-conjugated anti-IgG secondary antibody (antibody Info, cat.Immunolabeled cells were mounted for imaging, and a Zeiss LSM780 inverted microscope was used to acquire confocal images.

### Generation of circulating tumor cell-derived scRNA-seq data

To pick LM3-GFP cells from mouse mammary tissue and blood, single cells were sorted onto an HDM chip (Smart BiopsyTM Cell Isolator - CIS 030) and single cell RNA sequencing libraries were generated using the Chromium Single Cell 3' Library, Gel Bead & Multiplex kit and chip kit (10X Genomics), targeting 10,000 cells per library according to the manufacturer's conditions. This droplet-based system used barcodes (one for each cell) and Unique Molecular Identifiers (UMIs, one for each unique transcript) to obtain a unique 3'-mRNA gene expression profile from every captured cell. All samples were sequenced using an Illumina HiSeq4000 and mapped to the human reference genome (GRCh38) using Cell Ranger (10X Genomics).

### scRNA-seq bioinformatics for CDX models

Illumina outputs from 10X Genomics Chromium sequencing were processed using Cell Ranger 6.0.0. Data was exported from Cell Ranger to a Matrix Market format file and read in R using Seurat's Read 10X function. For each sample, an average of >135 million reads and 13,000 cells were obtained. Each 10X library was individually quality checked, and cells were filtered to ensure good gene coverage, consistency of read coverage, and low mitochondrial reads. For some libraries, the lower limit was reduced to 200 or 300, but in general, at least 500 genes were required to be detected in each cell. For mitochondrial reads, no more than 10% per cell was allowed, but the upper limit was sometimes increased to up to 65% and 92% for cells derived from primary tumor samples and blood samples, respectively. Cells with unusually high read or detected gene counts were filtered to minimize the generation of doublets (libraries generated from two cells). In addition, to prevent mouse-derived cells from contaminating the results, cells with more than 20% unmapped reads were filtered out. After performing the above quality filtering, 3,006 primary tumor sample-derived cells and 5,511 blood sample-derived cells were prepared for analysis. Statistical analysis of the 10X data was performed using the Seurat (v4.1.0, Seurat et al, 2019) software package for R. The data were analyzed using the following methods

Samples were combined by merging in Seurat, and cell clusters were identified by running the default Louvain clustering algorithm in Seurat. Default Seurat function settings were used, and a principal component dimension of 1:10 was used in all dimension reduction and integration steps. The cluster resolution value was set to 1.0 unless otherwise noted. RunUMAP Random Seed was set to 1,000 to ensure reproducibility. To detect genes not expressed in hemocytes, marker genes for cell clusters were identified using Seurat's FindAllMarkers function with default settings, excluding the percentage of cells expressing a particular gene.

### scRNA-seq bioinformatics for validation

The scRNA-seq data for ascites from ovarian cancer patients were retrieved from DUOS (Data Using Oversight System, Dataset ID: DUOS-000118). The scRNA-seq data of ascites from colorectal cancer patients was provided by H.S Kim, Department of Internal Medicine, Yonsei University College of Medicine. The scRNA-seq data of healthy PBMCs was provided by H.Y Gee, Department of Pharmacology, Yonsei University School of Medicine. All data were processed with the datat processing workflow of the CDX model, and circulating tumor cells were selected based on their epithelial cell marker expression scores using the celldex (v1.4.0, Liu et al, 2020) software package for R. The data were analyzed using the CDX model.

### Experimental Results

### Analyzing the limitations of the conventional biomarker EpCAM

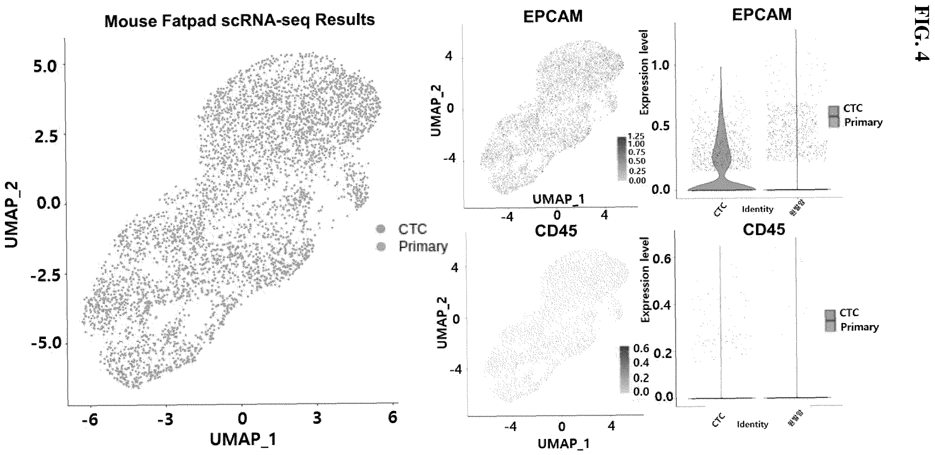

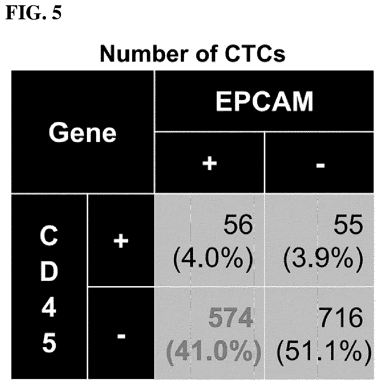

The expression of EpCAM in the mouse model was confirmed using single-cell transcriptome analysis and it was found that only 41.0% of CTCs expressed EpCAM and did not have CD45 (Figures 4 and 5). This result indicates that EpCAM is only detected in half of the CTCs, and that isolating CTCs based on EpCAM expression would miss a significant number of CTCs that do not express EpCAM. To overcome this limitation of EpCAM, the present inventors searched for a new biomarker of CTCs.

### Selection of biomarker candidates

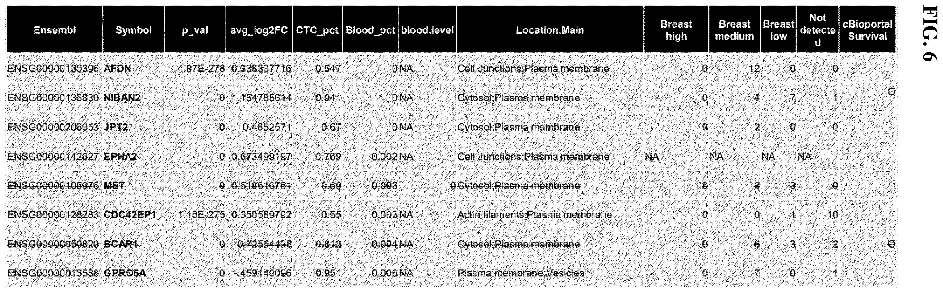

Comparative analysis of single-cell transcriptomes of previously isolated CTCs and blood revealed that RNA from eight of the genes encoding proteins located in the cell membrane (AFDN, BCAR1, CDC42EP1, EPHA2, GPRC5A, JPT2, MET, and NIBAN2) were exclusively expressed in CTCs (Figure 6). Of the eight genes mentioned, six cell membrane proteins were selected as candidate CTC biomarkers, excluding MET and BCAR1, which are well-known for their association with breast cancer aggravation.

The sequences of each of the genes AFDN, CDC42EP1, EPHA2, GPRC5A, JPT2, and NIBAN2 are attached hereto as Sequence Listings 1, 2, 3, 4, 5, and 6, respectively, and the sequences of the proteins encoded by the genes are attached hereto as Sequence Listings 7, 8, 9, 10, 11, and 12.

### Circulating Tumor Cells in Ovarian and Colorectal Cancer Patients

In addition to breast cancer, CTCs isolated from ascites of ovarian and colorectal cancer patients were also found to express six biomarkers, including AFDN, exclusively in blood.

### Potential as a biomarker

The above results confirm that RNA expression of AFDN, CDC42EP1, EPHA2, GPRC5A, JPT2, MET or NIBAN2 in cells in the bloodstream has potential as a biomarker for metastasis of several cancers.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. composition for diagnosing a cancer comprising, as an active ingredient, an agent for measuring the expression of at least one protein selected from the group consisting of AFDN, CDC42EP1, EPHA2, GPRC5A, JPT2 and NIBAN2, or a gene encoding the protein.

2. The composition of claim 1, wherein the cancer is a metastatic cancer.

3. The composition of claim 1, wherein the composition comprises an agent for measuring expression of the CDC42EP1 or NIBAN2 protein or the gene encoding the protein.

4. The composition of claim 1, wherein the agent for measuring the expression of a gene encoding the at least one protein selected from the group consisting of AFDN, CDC42EP1, EPHA2, GPRC5A, JPT2 and NIBAN2 is a primer or probe that specifically binds to a nucleic acid molecule of the gene.

5. The composition of claim 1, wherein the agent for measuring the expression of the at least one protein selected from the group consisting of AFDN, CDC42EP1, EPHA2, GPRC5A, JPT2, and NIBAN2 is an antibody or an antigen-binding fragment thereof that specifically binds to the at least one protein selected from the group consisting of AFDN, CDC42EP1, EPHA2, GPRC5A, JPT2 and NIBAN2; or an aptamer that specifically binds to the at least one protein selected from the group consisting of AFDN, CDC42EP1, EPHA2, GPRC5A, JPT2, and NIBAN2.

6. The composition of claim 1, wherein the cancer is breast cancer, ovarian cancer, or colon cancer.

7. A composition for detecting a Circulating Tumor Cell comprising, as an active ingredient, an agent for measuring the expression of at least one protein selected from the group consisting of AFDN, CDC42EP1, EPHA2, GPRC5A, JPT2 and NIBAN2, or a gene encoding the protein.

8. A kit for diagnosing a cancer comprising the composition of any one of claims 1 to 7.

9. The kit of claim 8, wherein the cancer is a metastatic cancer.

10. A method for providing information necessary for the diagnosis of cancer, comprising measuring the expression of at least one protein selected from the group consisting of AFDN, CDC42EP1, EPHA2, GPRC5A, JPT2 and NIBAN2, or genes encoding the protein, in a biological sample isolated from a subject.

11. The method of providing the information of claim 10, wherein the cancer is a metastatic cancer.

12. The method of claim 10, wherein the biological sample is selected from the group consisting of whole blood, plasma and serum.

13. The method of claim 10, wherein the cancer is breast cancer, ovarian cancer or colorectal cancer.

14. A composition for preventing or treating cancer metastasis comprising, as an active ingredient, an inhibitor of at least one protein selected from the group consisting of AFDN, CDC42EP1, EPHA2, GPRC5A, JPT2 and NIBAN2, or a gene encoding the protein.

15. A method for screening a composition for the preventing or treating cancer metastasis, comprising:
(a) contacting a test substance with a biological sample comprising at least one protein selected from the group consisting of AFDN, CDC42EP1, EPHA2, GPRC5A, JPT2 and NIBAN2, a gene encoding the protein, or a cell expressing the protein or gene; and
(b) measuring the expression of the at least one protein selected from the group consisting of AFDN, CDC42EP1, EPHA2, GPRC5A, JPT2 and NIBAN2, or the gene encoding the protein, in the biological sample,
wherein, when the expression level of the protein or the gene in the biological sample decreases, the test substance is determined to be a composition for preventing or treating cancer metastasis.

16. The method of claim 15, wherein the biological sample comprises cancer tissue or a cancer cell.
